# FASCICULE DE BREVET EUROPEEN

(11) **EP 1 207 875 B1**
(45) Date de publication et mention de la délivrance du brevet: **17.09.2003**
(21) Numéro de dépôt: 00952835.7
(22) Date de dépôt: 30.08.2000
(51) Int. Cl.: A61K 31/282, A61K 33/24, A61K 9/08, A61K 31/555

(54) **PREPARATION PHARMACEUTIQUE STABLE D'OXALIPLATINE POUR ADMINISTRATION PAR VOIE PARENTERALE**
OXALIPLATINHALTIGE PARENTERAL ANZUWENDENDE STABILISIERTE ARZNEIZUBEREITUNG UND VERFAHREN ZU DEREN HERSTELLUNG
PHARMACEUTICALLY STABLE OXALIPLATINUM PREPARATION FOR PARENTERAL ADMINISTRATION

(30) Priorité: 30.08.1999 US 151357 P
(43) Date de publication de la demande: 29.05.2002
(73) Titulaire: DEBIOPHARM S.A., 1000 Lausanne (CH)
(72) Inventeur: IBRAHIM, Houssam, CH-1255 Veyrier (CH); BAYSSAS, Martine, CH-1006 Lausannne (CH); POURRAT, Henri, F-63000 Clermont-Ferrand (FR); DEUSCHEL, Christine, CH-1270 Trélex (CH)
(74) Mandataire: Besse, François
(86) Numéro de dépôt international: CH0000462
(87) Numéro de publication internationale: WO01015691

(56) Documents cités:
- WO-A-94/12193
- WO-A-96/04904
- WO-A-98/39009
- WO-A-99/43355

## Description

La présente invention concerne une préparation pharmaceutique stable d'oxaliplatine, pour administration par voie parentérale destinée à être perfusée ou injectée, dans laquelle l'oxaliplatine se trouve en solution restant limpide, incolore et exempte de précipité après conservation à une gamme de températures allant de 2°C à 30°C pendant une durée pharmaceutiquement acceptable. L'invention concerne également un procédé de préparation de ladite préparation.

L'oxaliplatine (DCI, appelé également *l*-OHP), dérivé complexe du platine (CAS RN: 61825-94-3) décrit par Kidani et al. dans J. Med. Chem., 1978, 21, 1315, est un agent antinéoplasique utilisé par voie intraveineuse tout particulièrement dans le traitement des cancers colorectaux métastatiques. Actuellement, il est utilisé en milieu hospitalier sous une forme lyophilisée et sa préparation liquide est reconstituée à l'aide d'une solution glucosée juste avant son administration généralement en perfusion de courte durée.

L'oxaliplatine, sous cette forme lyophilisée, est formulée avec une quantité importante de lactose (d'un facteur 9 en poids par rapport à l'oxaliplatine). C'est alors une poudre ou un gâteau de couleur blanchâtre. Lors de sa reconstitution, il est préconisé d'utiliser une quantité de solution glucosée telle que la concentration en oxaliplatine dans la préparation ainsi obtenue soit comprise entre 2,5 et 5,0 mg/ml.

L'oxaliplatine, sous sa forme de substance active pure, est connu pour être légèrement soluble dans l'eau, très peu soluble dans le méthanol et pratiquement insoluble dans l'éthanol et l'acétone. Plus précisément, la solubilité maximale de l'oxaliplatine à saturation dans l'eau à 37°C est de 7,9 mg/ml, mais à 20°C, elle chute à 6 mg/ml. Dans le méthanol à 20°C, elle n'est plus que de 0,22 mg/ml.

Récemment, une préparation d'oxaliplatine pharmaceutiquement stable, prête à être administrée par voie parentérale en perfusion, constituée par une solution aqueuse d'oxaliplatine à une concentration d'environ 2 mg/ml, et ne contenant pas d'autres adjuvants, a été décrite par Ibrahim et al. dans WO 96/04904.

Cette préparation offrait au personnel hospitalier le grand avantage, d'une part, de ne plus à avoir à manipuler une poudre ou un gâteau cytotoxique lors de la reconstitution de la préparation pharmaceutique et, d'autre part, d'éviter tout risque d'utiliser par erreur une solution de reconstitution contenant des ions chlorure, telle qu'une solution de chlorure de sodium habituellement utilisée dans ce genre d'opération, qui a pour conséquence grave de décomposer le complexe métallique.

En revanche, cette préparation n'était pas satisfaisante de fait, en particulier, de sa concentration en oxaliplatine très inférieure aux solubilités mentionnées ci-dessus. Cette faible concentration est requise pour prévenir de tout risque de formation de précipités ou de cristaux susceptibles d'apparaître, par exemple, au cours d'une conservation aux basses températures d'un réfrigérateur ou lors d'un transport dans des conditions hivernales. Lorsque de tels précipités apparaissent dans une préparation pharmaceutique, le personnel hospitalier est généralement averti de mettre à l'écart, dans le doute, l'échantillon. Si toutefois, une tentative de redissolution était entreprise, une opération de chauffage à des températures supérieures à 40°C, couplée éventuellement avec une opération de sonification, devrait être entreprise.

De ce fait, une préparation pharmaceutique à base d'une solution d'oxaliplatine à une concentration de 2 mg/ml, telle que décrite dans WO 96/04904, nécessite la manipulation de grands volumes. A titre indicatif, la posologie généralement recommandée lors d'un traitement mettant en oeuvre une perfusion courte durant entre 2 et 6 heures est de 130 mg d'oxaliplatine par m² de surface corporelle. En prenant comme surface corporelle moyenne une valeur de 1,7 m², il convient alors d'utiliser au moins 110 ml de cette préparation à 2 mg/ml.

L'un des buts de la présente invention est de mettre à disposition une préparation pharmaceutique stable d'oxaliplatine, pour administration par voie parentérale destinée à être perfusée ou injectée, dont la concentration en oxaliplatine serait nettement augmentée de façon à réduire de façon importante les volumes à manipuler et/ou à utiliser.

Avec de telles préparations, il sera alors possible de faciliter encore le travail du personnel hospitalier en améliorant d'autant leur sécurité.

En effet, le nombre de flacons, ou leur volume, sera plus faible, réduisant ainsi les risques lorsqu'une mauvaise manipulation entraînerait leur casse. De plus, le volume requis pour une perfusion ou une injection devenant plus faible, il sera alors possible d'utiliser des seringues dites préremplies de tailles disponibles dans le commerce, ce qui évitera toute manipulation de transvasement devant être effectuée dans des conditions aseptiques dans la pharmacie de l'hôpital. L'adjonction d'un dispositif d'actionnement du piston de la seringue tel qu'un pousse seringue permettra le contrôle à volonté du débit au cours de la perfusion. Un autre avantage sera de pouvoir fournir de telles préparations dans des flacons dits multidoses contenant alors un plus grand nombre de doses et permettant au praticien de prélever à volonté le volume voulu de la préparation pharmaceutique d'oxaliplatine sans avoir à mettre à l'écart la partie résiduelle de la préparation non utilisée.

Un autre des buts de la présente invention est de mettre à disposition une préparation pharmaceutique d'oxaliplatine pour administration par voie parentérale à concentration élevée, qui soit stable pendant une durée pharmaceutiquement acceptable, c'est à dire qui reste limpide, incolore et exempte de tout précipité à une gamme de températures allant de 2°C à 30°C susceptibles d'être rencontrées au cours de son transport, de son stockage et/ou de sa manipulation.

A cet effet, il a été trouvé, de façon surprenante, que, du fait de la présence au sein d'une préparation pharmaceutique d'oxaliplatine d'un nombre très limité de dérivés hydroxylés, dont l'utilisation est généralement acceptée pour la préparation de médicaments, la concentration en oxaliplatine et sa stabilité dans un domaine large de températures d'utilisation s'en trouvaient améliorées de façon importante.

Ainsi, l'un des objets de la présente invention est une préparation pharmaceutique stable d'oxaliplatine pour administration par voie parentérale, l'oxaliplatine se trouvant en solution dans un solvant à une concentration d'au moins 7 mg/ml et le solvant comprenant une quantité suffisante d'au moins un dérivé hydroxylé choisi parmi les 1,2-propanediol, glycérol, maltitol, saccharose et inositol. De préférence, l'oxaliplatine se trouve en solution dans un solvant à une concentration d'au moins 7,5 mg/ml.

Par préparation pharmaceutique stable d'oxaliplatine pour administration par voie parentérale, on entend une préparation liquide répondant aux critères généralement fixés par les autorités de santé afin qu'elle soit susceptible d'être administrée par voie parentérale chez le mammifère. Parmi ces critères respectés, cette préparation est limpide, incolore et exempte de précipité et le reste ainsi pendant une durée pharmaceutiquement acceptable d'au moins 6 mois, cette durée allant même jusqu'au moins 3 ans, en étant manipulée et/ou conservée à des températures pouvant varier entre environ 2°C et environ 30°C.

Le choix limité des dérivés hydroxylés à utiliser s'est fait à la suite d'un très grand nombre d'essais de substances habituellement reconnues pour améliorer la solubilité en milieu aqueux de substances médicamenteuses. Certains des ces essais apparaîtront, à titre comparatif, dans les exemples. A titre indicatif, mélangés à l'eau, des alcools tels que l'éthanol et l'alcool benzylique, la diméthylformamide ou le diméthylacétamide n'ont pas permis d'augmenter sensiblement la solubilité de l'oxaliplatine. Parmi les polyalkylène et en particulier les polyéthylène glycols ayant une masse moléculaire allant de 150 à 6000, seul le polyéthylène glycol a permis d'augmenter de façon importante la solubilité de l'oxaliplatine. Ce composé n'a toutefois pas été retenu en tant que composant possible du solvant du fait que la solution obtenue était très colorée. Les éthers couronne tels que certaines cyclodextrines ont permis d'augmenter très légèrement la concentration de l'oxaliplatine mais pas suffisamment pour les applications désirées. Parmi les hydrates de carbone solubilisés dans l'eau, le lactose, le sorbitol, le solkétal, le mannitol, entre autres, se sont montrés inefficaces. D'autres hydrates de carbone tels que cellobiose, le tréhalose, le mélibiose, le gentiobiose, le raffinose, le stachyose ou le mélozitose ont montré que, solubilisés dans l'eau, ils permettaient de dissoudre, au moins en partie, l'oxaliplatine mais ils étaient disponibles dans le commerce à un prix prohibitif pour être utilisés comme solvant. Une large gamme de tensioactifs, en particulier pour le Tween 20, le Tween 60 et le Tween 80 se sont montrés inefficaces pour solubiliser l'oxaliplatine.

De préférence, dans la préparation pharmaceutique selon l'invention, l'oxaliplatine se trouve en solution dans le solvant à une concentration d'au moins 9 mg/ml. Dans ce cas, un millilitre du solvant comprend au moins 100 mg d'un ou plusieurs des dérivés hydroxylés choisi parmi les 1,2-propane-diol, glycérol, maltitol, saccharose et inositol. Lorsque les dérivés hydroxylés se trouvent à l'état liquide à température ambiante, alors le solvant peut être constitué à 100% d'au moins un de ces dérivés liquides. En général, le solvant comprend également de l'eau. L'eau utilisée est de préférence de l'eau telle que définie par la Pharmacopée européenne comme étant de l'eau pour préparations injectables.

De préférence encore, l'oxaliplatine se trouve en solution dans le solvant à une concentration comprise entre environ 10 mg/ml et environ 15 mg/ml.

Le solvant de la préparation selon l'invention peut en outre comprendre d'autres composés, excipients, adjuvants ou additifs, habituellement préconisés par la Pharmacopée européenne lors de la préparation de préparations pharmaceutiques parentérales, à l'exception en particulier de tout composé, complexe métallique ou sel, susceptible de dégrader chimiquement l'oxaliplatine. Cela est le cas pour des composés, des complexes métalliques générant, en particulier au contact de l'eau, des ions chlorure, ou des sels contenant des ions chlorure comme le chlorure de sodium habituellement utilisé pour assurer l'isotonie.

Ainsi, il est possible d'utiliser un tampon pour contrôler le pH de la préparation et/ou renforcer la stabilité de l'oxaliplatine. Toutefois, cet usage ne s'est pas avéré indispensable. Si cependant l'on utilise un tampon, alors ce doit être un tampon contenant au moins l'un des ligands du complexe métallique de l'oxaliplatine ou les précurseurs de ce ligand, par exemple un tampon à base en particulier d'acide oxalique ou l'un de ses sels tel que l'oxalate de sodium, de préférence l'oxalate de sodium.

Il est également possible, pour assurer l'isotonie au sang de la préparation, d'utiliser, par exemple, une quantité appropriée de glucose.

Il est également possible, pour assurer une action antimicrobienne, d'utiliser des conservateurs antimicrobiens. Toutefois, cet usage ne s'est pas avéré indispensable du fait que l'oxaliplatine en solution a présenté lui-même une efficacité antimicrobienne dans un essai par contamination artificielle préconisé par la Pharmacopée européenne.

De préférence, la préparation pharmaceutique d'oxaliplatine selon l'invention est conditionnée dans un récipient scellable ou hermétique approprié pour une administration parentérale. Un tel récipient peut être par exemple un flacon dit multidoses, une seringue dite préremplie, une poche souple à perfusion ou une ampoule.

Le flacon multidoses est généralement muni d'un bouchon septum autorisant le passage d'une aiguille de seringue et renferme une quantité de la préparation selon l'invention, prélevable à volonté, suffisante pour permettre de mettre en oeuvre un certain nombre de perfusions ou d'injections. A titre indicatif, un flacon multidoses de 500 ml renfermant la préparation selon l'invention dans laquelle l'oxaliplatine se trouve à une concentration de 10 mg/ml contient une quantité suffisante de préparation pour mettre en oeuvre une vingtaine de perfusions ou d'injections. Comme mentionné ci-dessus, la préparation selon l'invention a montré en elle-même des propriétés antimicrobiennes telles que l'ajout d'aucun agent de conservation n'est pas nécessaire.

La seringue préremplie présente l'avantage qu'aucun transvasement à l'air libre de la préparation selon l'invention n'est nécessaire, et donc il n'est plus obligé de préparer l'équipement d'injection et/ou de perfusion en conditions aseptiques dans la pharmacie de l'hôpital parfois éloignée de la chambre de traitement. Le volume maximum des seringues préremplies disponible dans le commerce est généralement de 50 ml. Comme indiqué ci-dessus, la préparation connue de l'art antérieur a une concentration en oxaliplatine trop faible pour qu'une seule seringue préremplie d'un volume de 50 ml puisse être utilisée lors d'une même perfusion. A titre indicatif, la quantité de la préparation selon l'invention ayant une concentration en oxaliplatine de 10 mg/ml nécessaire lors d'un traitement par perfusion courte peut être contenue dans une seringue préremplie de 25 ml. Avec une telle seringue de 25 ml, il est alors également possible d'utiliser par exemple un pousse-seringue à vitesse programmable permettant ainsi de contrôler de façon fiable le débit au cours de la perfusion ou de l'injection, ce qui allège d'autant le travail de surveillance du praticien.

Un des autres objets de la présente invention est l'utilisation, pour la manipulation et/ou le stockage et/ou l'administration de la préparation pharmaceutique, d'un flacon dit multidoses, d'une seringue dite préremplie, d'une poche souple à perfusion ou d'une ampoule.

Un autre des objets de la présente invention est un procédé de préparation d'une préparation pharmaceutique d'oxaliplatine telle mentionnée ci-dessus, comprenant une étape de mise en solution de l'oxaliplatine avec un solvant comprenant une quantité suffisante d'au moins un dérivé hydroxylé choisi parmi les 1,2-propane-diol, glycérol, maltitol, saccharose et inositol.

Plus précisément, ce procédé comprend les étapes suivantes:
a) la mise en contact à une température inférieure à 80°C d'une quantité d'oxaliplatine avec une quantité suffisante dudit solvant afin d'obtenir une concentration en oxaliplatine d'au moins 7 mg/ml;
b) l'établissement du mélange obtenu à l'étape a) à une température comprise entre 15°C et 30°C;
c) la soumission du mélange obtenu à l'étape b) à une étape de stérilisation; et
d) la conservation dans un récipient approprié tel que mentionné ci-dessus pour une administration parentérale du mélange obtenu à l'étape c) à une température comprise entre 2°C et 30°C.

De préférence, la mise en contact selon l'étape a) se fait à une température comprise entre 20°C et 60°C. De préférence encore, elle se fait à une température comprise entre 30°C et 60°C. L'opération de stérilisation selon l'étape c) se fait selon les méthodes habituelles bien connues du spécialiste.

Les préparations pharmaceutiques selon l'invention particulièrement intéressantes, leur mode de préparation, leurs avantages en particulier la façon de les mettre en oeuvre sont décrits dans les exemples qui suivent.

### Exemple 1: Préparation des préparations pharmaceutiques et essais de solubilité à une concentration de 10 mg/ml

Le choix des composés susceptibles d'être compris dans le solvant de la préparation selon l'invention s'est fait après observation visuelle d'une solution limpide incolore contenant 10 mg d'oxaliplatine par ml des solvants respectifs après deux périodes d'agitations de 24 heures à 25°C.

Pour chacun des mélanges observés, les classements visuels suivants ont été attribués:
- insoluble (I): la quantité solide initiale d'oxaliplatine est restée pratiquement inchangée;
- partiellement soluble (PS): la quantité solide initiale d'oxaliplatine a été diminuée significativement; et
- soluble (S): la quantité solide initiale d'oxaliplatine a été entièrement ou pratiquement entièrement dissoute.
L'apparition d'une coloration (C) a également été observée.

Une poudre d'oxaliplatine (250 mg) est placée dans un récipient de 50 ml jaugé à 25 ml. Le composé à sélectionner (quantité mentionnée dans le tableau 1) est introduit selon dans le récipient conjointement ou préalablement à une addition d'eau en complément à 25 ml.

**Tableau 1**

| No du mélange | 1.1 | 1.2 | 1.3 | 1.4 | 1.5 | 1.6 |
|---|---|---|---|---|---|---|
| Composé | éthanol | 1,2-propanediol | glycérol | lactose | maltitol | sorbitol |
| Quantité | 9,8 g (12,5 ml) | 12,95 g (12,5 ml) | 15,76 g (12,5 ml) | 1,25 g | 5 g | 10 g |
| Solubilité | PS | S | S | PS | S | PS |
| Coloration | - | - | - | - | - | - |
| No du mélange | 1.7 | 1.8 | 1.9 | 1.10 | 1.11 | 1.12 |
| Composé | saccharose | inositol | PEG 200 | PEG 300 | Tween 20 | Tween 80 |
| Quantité | 2,5 g | 2,5 g | 14 g (12,5 ml) | 14 g (12,5 ml) | 27,4 g (25 ml) | 26,6 g (25 ml) |
| Solubilité | S | S | S | PS | PS | PS |
| Coloration | - | - | C jaunâtre | C jaunâtre | C | C |

Des observations consignées dans le tableau 1, il ressort que, à 25°C sous deux agitations de 24 heures chacune, 250 mg d'oxaliplatine dans 250 ml des mélanges No. 1.2, 1.3, 1.5, 1.7 et 1.8 apparaît comme étant soluble et que les solutions obtenues sont incolores. Ces mélanges sont constitués d'eau et respectivement de 1,2-propanediol, de glycérol, de maltitol, de saccharose et d'inositol.

Dans les autres mélanges mentionnés, il est, soit soluble mais dans ce cas conduit à des solutions colorées jaunâtre, soit partiellement soluble. Les nombreux autres composés constituants des mélanges dans lesquels l'oxaliplatine s'est montré insolubles, soit très partiellement soluble, n'ont pas été consignés dans ce tableau.

### Exemple 2: Optimisation de la composition du solvant pour une concentration de 10 mg/ml

Une optimisation de la quantité des composés susceptibles d'être compris dans le solvant de la préparation selon l'invention s'est faite après observation visuelle d'une solution limpide incolore contenant 10 mg d'oxaliplatine par ml des solvants respectifs après deux périodes d'agitations de 24 heures à 25°C, de façon similaire à l'Exemple 1 avec cette fois encore 250 mg d'oxaliplatine dans un récipient de 50 ml jaugé à 25 ml et complété à 25 ml par le solvant respectif.

Le tableau 2 consigne, pour les composés hydroxylés 1,2-propanediol, glycérol et maltitol, les mélanges dans lesquels aucun résidu n'est décelable et les mélanges dans lesquels les premiers résidus apparaissent.

**Tableau 2**

| No du mélange | 2.1 | 2.2 | 2.3 | 2.4 | 2.5 | 2.6 |
|---|---|---|---|---|---|---|
| Composé | 1,2-propanediol | 1,2-propanediol | glycérol | glycérol | maltitol | maltitol |
| Quantité | 5,9 g (7,5 ml) | 3,9 g (5,0 ml) | 6,3 g (5,0 ml) | 3,1 g (2,5 ml) | 5 g | 0,5 g |
| Solubilité | S | PS | S | PS | S | PS |

### Exemple 3: Optimisation de la composition du solvant pour une concentration supérieure à 10 mg/ml

Une optimisation de la quantité des composés hydroxylé compris dans le solvant de la préparation selon l'invention s'est faite après observation visuelle d'une solution limpide incolore en augmentant progressivement à la fois la quantité d'oxaliplatine et la quantité du composé hydroxylé à volume constant de solvant, en suivant toujours le protocole décrit dans l'Exemple 1.

Ainsi, pour un volume complété à 25 ml, un mélange aqueux contenant 337,5 mg d'oxaliplatine et 5 g de maltitol est limpide. En revanche, des résidus apparaissent dans un mélange aqueux contenant 400 mg d'oxaliplatine et 12,5 g de maltitol.

L'échantillon contenant les 337,5 mg d'oxaliplatine et le 5 g de maltitol a été soumis à des variations de températures qui pourraient être qualifiées d'extrêmes. Tout d'abord, il a été porté à 60°C et a été maintenu à cette température sous agitation pendant une heure. La solution ainsi obtenue était limpide et incolore. Elle a été soumise ensuite à trois congélations successives avec, dans l'intervalle, retour à température ambiante. Cette solution a ensuite séjourné pendant 7 jours à l'intérieur d'un réfrigérateur. A la suite de ce traitement, après retour à la température ambiante, aucun cristal n'a été observé.

Dans 25 ml de glycérol à 85%, il a été possible de dissoudre en partie 379,7 d'oxaliplatine.

### Exemple 4: Détermination de la solubilité maximale de l'oxaliplatine à température ambiante (21±2°C) et à température du réfrigérateur (5±3°C)

Pour conduire cette détermination, cinq solvants ont été préparés. Ils ont les compositions suivantes:
- solvant 4.1: 1,2-propanediol (50 ml) et eau (50 ml);
- solvant 4.2: glycérol à 85% (50 ml) et eau (50 ml);
- solvant 4.3: glycérol à 85% (40 ml) et eau (60 ml);
- solvant 4.4:1,2-propanediol (25 ml), glycérol (25 ml) et eau (50 ml); et
- solvant 4.5: maltitol (50 g) et eau (100 ml).

Pour chaque mélange à examiner, l'oxaliplatine (1 g) et le solvant à considérer (50 ml) sont introduits dans un erlenmeyer de 100 ml. Le mélange est placé dans une étuve à une température de 40°C et est soumis à une agitation pendant 120 minutes. Des prélèvements d'échantillon sont effectués respectivement à 90 minutes et à 120 minutes. Une partie de ces échantillons sont amenés à température ambiante (21±2°C). Un contrôle visuel est effectué puis les échantillons sont alors filtrés. La teneur en oxaliplatine (mg/ml) est analysée quantitativement par chromatographie HPLC selon des paramètres bien établis. Tous les résultats sont consignés dans le tableau 4.

**Tableau 4**

| No. Mélange | Aspect | Teneur en oxaliplatine (mg/ml),T_{90 min}, 21±2°C | Teneur en oxaliplatine (mg/ml), T_{120 min}, 21±2°C |
|---|---|---|---|
| 4.1 | incolore | 14,01 | 14,33 |
| 4.2 | incolore | 13,59 | 13,68 |
| 4.3 | incolore | 12,77 | 12,93 |
| 4.4 | incolore | 13,74 | 13,70 |
| 4.5 | incolore | 13,04 | 13,14 |

Une autre partie des échantillons contenant le mélange 4.5 à base du solvant 4.5 sont placés à l'intérieur d'un réfrigérateur à une température de 5±3°C puis laissés à cette température pendant 7 jours. Un contrôle visuel est effectué puis les échantillons sont alors filtrés à froid. La teneur en oxaliplatine (mg/ml) est analysée quantitativement par chromatographie HPLC selon des paramètres bien établis. La teneur mesurée en oxaliplatine est de 12,84 mg/ml et la solution est incolore.

### Exemple 5: Contrôle de la stabilité sur 3 mois

Pour conduire ce contrôle, l'oxaliplatine à été dissout à raison de 10 mg/ml dans respectivement les quatre solvants de compositions suivantes:
- solvant 5.1:1,2-propanediol (50 ml) et eau PPI (50 ml);
- solvant 5.2: glycérol à 85% (50 ml) et eau PPI (50 ml);
- solvant 5.4:1,2-propanediol (25 ml), glycérol (25 ml) et eau PPI (50 ml); et
- solvant 5.5: maltitol (50 g) et eau PPI (100 ml);
avec l'expression "eau PPI" signifiant Eau pour préparations injectables au sens de la Pharmacopée européenne.

Les préparations ainsi obtenues, réparties en un certain nombre de lots, ont été stérilisées selon les méthodes habituelles connues du spécialiste. Ces lots ont été conservés à l'abri de la lumière pendant une durée de 3 mois, une première partie à une température d'environ 25°c à un taux d'humidité relative de 60%, une deuxième partie à une température d'environ 40°c à un taux d'humidité relative de 75% et enfin une troisième partie à une température d'environ 4°C. Des prélèvements ont été effectués au temps 0, à 1 mois puis à 3 mois, et ont été soumis à un certains nombres d'analyses physico-chimiques. Les résultats obtenus ont montré que les quatre préparations sont stables sur une durée allant au moins jusqu'à 3 mois.

### Exemple 6: Efficacité de la conservation antimicrobienne

Cette étude a été conduite selon la méthode préconisée à la section 5.1.3. de la Pharmacopée européenne ayant pour titre "Efficacité de la conservation antimicrobienne" en exposant une préparation d'oxaliplatine à la souche *Staphyloccus aureus.* Sous ces conditions, les résultats obtenus ont montré une nette réduction de la population de *Staphyloccus aureus* avec un Δlog de 5,99 après 24 heures.

## Revendications

1. Préparation pharmaceutique stable d'oxaliplatine pour administration par voie parentérale, **caractérisée en ce que** l'oxaliplatine se trouve en solution dans un solvant à une concentration d'au moins 7 mg/ml et **en ce que** ledit solvant comprend une quantité suffisante d'au moins un dérivé hydroxylé choisi parmi les 1,2-propanediol, glycérol, maltitol, saccharose et inositol.

2. Préparation pharmaceutique selon la revendication 1, **caractérisée en ce que** l'oxaliplatine se trouve en solution dans ledit solvant à une concentration d'au moins 9 mg/ml et **en ce que** 1 ml dudit solvant comprend au moins 100 mg d'un ou plusieurs desdits dérivés hydroxylés.

3. Préparation pharmaceutique selon la revendication 2, **caractérisée** ce que ledit solvant comprend en outre de l'eau.

4. Préparation pharmaceutique selon la revendication 3, **caractérisée en ce que** l'oxaliplatine se trouve en solution dans ledit solvant à une concentration comprise entre environ 10 mg/ml et environ 15 mg/ml.

5. Préparation pharmaceutique selon l'une quelconque des revendications précédentes, **caractérisée en ce qu'**elle se trouve conditionnée dans un récipient approprié pour une administration parentérale.

6. Préparation pharmaceutique selon la revendication 5, **caractérisé en ce que** ledit récipient est un flacon multidoses.

7. Préparation pharmaceutique selon la revendication 5, **caractérisé en ce que** ledit récipient est une seringue dite préremplie.

8. Préparation pharmaceutique selon la revendication 5, **caractérisé en ce que** ledit récipient est une poche souple à perfusion.

9. Préparation pharmaceutique selon la revendication 5, **caractérisé en ce que** ledit récipient est une ampoule.

10. Procédé de préparation d'une préparation pharmaceutique selon l'une quelconque des revendications précédentes comprenant une étape de mélange de l'oxaliplatine avec un solvant comprenant une quantité suffisante d'au moins un dérivé hydroxylé choisi parmi les 1,2-propane-diol, glycérol, maltitol, saccharose et inositol.

11. Procédé selon la revendication 10, **caractérisé en ce qu'**il comprend les étapes suivantes:
a) mettre en contact à une température inférieure à 80°C une quantité d'oxaliplatine avec une quantité suffisante dudit solvant afin d'obtenir une concentration en oxaliplatine d'au moins 7 mg/ml;
b) ramener le mélange obtenu à l'étape a) à une température comprise entre 15°C et 30°C;
c) soumettre le mélange obtenu à l'étape b) à une filtration aseptique; et
d) conserver dans un récipient approprié pour une administration parentérale le mélange obtenu à l'étape c) à une température comprise entre 2°C et 30°C.

12. Utilisation d'un flacon multidoses pour conserver la préparation pharmaceutique selon l'une quelconque des revendications 1 à 4.

13. Utilisation d'une seringue dite préremplie pour conserver et/ou manipuler la préparation pharmaceutique selon l'une quelconque des revendications 1 à 4.

14. Utilisation d'une poche souple à perfusion pour conserver et/ou manipuler la préparation pharmaceutique selon l'une quelconque des revendications 1 à 4.

## Claims

1. Stable oxaliplatin pharmaceutical preparation for administration by the parenteral route, **characterized in that** the oxaliplatin is in solution in a solvent at a concentration of at least 7 mg/ml and **in that** the said solvent comprises a sufficient amount of at least one hydroxylated derivative chosen from 1,2-propanediol, glycerol, maltitol, sucrose and inositol.

2. Pharmaceutical preparation according to Claim 1, **characterized in that** the oxaliplatin is in solution in the said solvent at a concentration of at least 9 mg/ml and **in that** 1 ml of the said solvent comprises at least 100 mg of one or more of the said hydroxylated derivatives.

3. Pharmaceutical preparation according to Claim 2, **characterized in that** the said solvent additionally comprises water.

4. Pharmaceutical preparation according to Claim 3, **characterized in that** the oxaliplatin is in solution in the said solvent at a concentration of between approximately 10 mg/ml and approximately 15 mg/ml.

5. Pharmaceutical preparation according to any one of the preceding claims, **characterized in that** it is packaged in a container appropriate for parenteral administration.

6. Pharmaceutical preparation according to Claim 5, **characterized in that** the said container is a multidose bottle.

7. Pharmaceutical preparation according to Claim 5, **characterized in that** the said container is a "prefilled" syringe.

8. Pharmaceutical preparation according to Claim 5, **characterized in that** the said container is a flexible infusion bag.

9. Pharmaceutical preparation according to Claim 5, **characterized in that** the said container is a vial.

10. Process for the preparation of a pharmaceutical preparation according to any one of the preceding claims, comprising a stage of mixing the oxaliplatin with a solvent comprising a sufficient amount of at least one hydroxylated derivative chosen from 1,2-propanediol, glycerol, maltitol, sucrose and inositol.

11. Process according to Claim 10, **characterized in that** it comprises the following stages:
a) bringing an amount of oxaliplatin and a sufficient amount of the said solvent into contact at a temperature of less than 80°C in order to obtain a concentration of oxaliplatin of at least 7 mg/ml;
b) bringing the mixture obtained in stage a) back to a temperature of between 15°C and 30°C;
c) subjecting the mixture obtained in stage b) to aseptic filtration; and
d) storing the mixture obtained in stage c) at a temperature of between 2°C and 30°C in a container appropriate for parenteral administration.

12. Use of a multidose bottle for storing the pharmaceutical preparation according to any one of Claims 1 to 4.

13. Use of a "prefilled" syringe for storing and/or handling the pharmaceutical preparation according to any one of Claims 1 to 4.

14. Use of a flexible infusion bag for storing and/or handling the pharmaceutical preparation according to any one of Claims 1 to 4.

## Patentansprüche

1. Stabiles pharmazeutisches Oxaliplatin-Präparat zur parenteralen Verabreichung, **dadurch gekennzeichnet, daß** das Oxaliplatin in einer Konzentration von mindestens 7 mg/ml gelöst in einem Lösungsmittel vorliegt und daß das Lösungsmittel eine ausreichende Menge an mindestens einem Hydroxyderivat aus der Gruppe 1,2-Propandiol, Glycerin, Maltit, Saccharose und Inosit enthält.

2. Pharmazeutisches Präparat nach Anspruch 1, **dadurch gekennzeichnet, daß** das Oxaliplatin in einer Konzentration von mindestens 9 mg/ml gelöst in dem Lösungsmittel vorliegt und daß 1 ml des Lösungsmittels mindestens 100 mg an einem oder mehreren der Hydroxyderivate enthält.

3. Pharmazeutisches Präparat nach Anspruch 2, **dadurch gekennzeichnet, daß** das Lösungsmittel weiterhin Wasser enthält.

4. Pharmazeutisches Präparat nach Anspruch 3, **dadurch gekennzeichnet, daß** das Oxaliplatin in einer Konzentration zwischen ungefähr 10 mg/ml und ungefähr 15 mg/ml gelöst in dem Lösungsmittel vorliegt.

5. Pharmazeutisches Präparat nach einem der vorhergehenden Ansprüche, **dadurch gekennzeichnet, daß** es in einem für die parenterale Verabreichung geeigneten Behältnis verpackt ist.

6. Pharmazeutisches Präparat nach Anspruch 5, **dadurch gekennzeichnet, daß** es sich bei dem Behältnis um eine Mehrdosenflasche handelt.

7. Pharmazeutisches Präparat nach Anspruch 5, **dadurch gekennzeichnet, daß** es sich bei dem Behältnis um eine Fertigspritze handelt.

8. Pharmazeutisches Präparat nach Anspruch 5, **dadurch gekennzeichnet, daß** es sich bei dem Behältnis um einen Perfusionsbeutel handelt.

9. Pharmazeutisches Präparat nach Anspruch 5, **dadurch gekennzeichnet, daß** es sich bei dem Behältnis um eine Ampulle handelt.

10. Verfahren zur Herstellung eines pharmazeutischen Präparats nach einem der vorhergehenden Ansprüche, umfassend den Schritt, daß man das Oxaliplatin mit einem Lösungsmittel, das eine ausreichende Menge an mindestens einem Hydroxyderivat aus der Gruppe 1,2-Propandiol, Glycerin, Maltit, Saccharose und Inosit enthält, mischt.

11. Verfahren nach Anspruch 10, **dadurch gekennzeichnet, daß** es die folgenden Schritte umfaßt:
a) Inkontaktbringen einer Menge an Oxaliplatin mit einer ausreichenden Menge des Lösungsmittels bei einer Temperatur unter 80°C, um zu einer Oxaliplatinkonzentration von mindestens 7 mg/ml zu gelangen;
b) Erwärmen der in Schritt a) erhaltenen Mischung auf eine Temperatur zwischen 15°C und 30°C;
c) Sterilfiltrieren der in Schritt b) erhaltenen Mischung; sowie
d) Aufbewahren der in Schritt c) erhaltenen Mischung in einem zur parenteralen Verabreichung geeigneten Behältnis bei einer Temperatur zwischen 2°C und 30°C.

12. Verwendung einer Mehrdosenflasche zur Aufbewahrung des pharmazeutischen Präparats nach einem der Ansprüche 1 bis 4.

13. Verwendung einer Fertigspritze zur Aufbewahrung und/oder Handhabung des pharmazeutischen Präparats nach einem der Ansprüche 1 bis 4.

14. Verwendung eines Infusionsbeutels zur Aufbewahrung und/oder Handhabung des pharmazeutischen Präparats nach einem der Ansprüche 1 bis 4.
